# EUROPEAN PATENT APPLICATION

(11) **EP 1 437 130 A1**
(43) Date of publication of application: **14.07.2004**
(21) Application number: 02777864.6
(22) Date of filing: 17.10.2002
(51) Int. Cl.: A61K 9/70, A61K 31/445, A61K 47/12, A61K 47/14, A61K 47/16, A61K 47/34, A61P 25/28

(54) **PERCUTANEOUS ABSORPTION PREPARATIONS**

(30) Priority: 17.10.2001 JP 2001319510
(71) Applicant: HISAMITSU PHARMACEUTICAL CO. INC., Tosu-shi, Saga-ken 841-0017 (JP)
(72) Inventor: TERAHARA, Takaaki, Tsukuba R & D Center, Tsukuba-shi, Ibaraki 305-0856 (JP); TOSHIMITSU, Arata, Tsukuba R & D Center, Tsukuba-shi, Ibaraki 305-0856 (JP); UEMURA, Kengo, Tsukuba R & D Center, Tsukuba-shi, Ibaraki 305-0856 (JP); HIGO, Naruhito, Tsukuba R & D Center, Tsukuba-shi, Ibaraki 305-0856 (JP); GOTO, Takeshi, Tsukuba R & D Center, Tsukuba-shi, Ibaraki 305-0856 (JP); SATO, Shuji, Tsukuba R & D Center, Tsukuba-shi, Ibaraki 305-0856 (JP)
(74) Representative: Schnappauf, Georg Dr.
(86) International application number: PCT/JP2002/010785
(87) International publication number: WO 2003/032960

(57) **Abstract**

Percutaneous absorption preparations for treating dementia which contain an adhesive composition, characterized in that the adhesive composition contains the active ingredient in a dispersed state, the active ingredient is released at a pharmacologically effective rate and the skin permeation rate thereof is at least 1.2 µg/cm²/h. Thus, it is possible to provide percutaneous absorption preparations whereby the therapeutic effect can be sustained over a prolonged period of time without elevating the concentration of the active ingredient in the plasma to such a level as causing the expression of side effects in the administration of remedies for dementia.

## Description

### Technical Field

The present invention relates to a percutaneous absorption preparation for the treatment of dementia and, in particular, to a percutaneous absorption preparation of Donepezil, Icopezil, Zanapezil, ER-127528, etc., which are drugs for the treatment of Alzheimer's dementia.

### Background Art

Donepezil is an acetylcholinesterase (AChE) inhibitor, and is currently used clinically as a drug for the treatment of Alzheimer's dementia. In Alzheimer's dementia, in which a disorder in the intracranial cholinergic nervous system has been reported, an AChE inhibitor such as Donepezil increases the intracranial acetylcholine and activates the intracranial cholinergic nervous system. Donepezil preparations that are currently used in practice are tablets, and are prescribed for Alzheimer's dementia patients as oral preparations.

Furthermore, as a compound that has similar AChE inhibition and is currently under development as a drug for the treatment of Alzheimer's dementia, ER-127528, which is represented by the formula below, 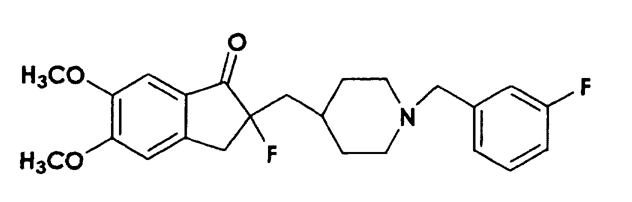 Icopezil, Zanapezil, etc. can be cited. With regard to ER-127528, JP, A, 2000-319258 discloses that this compound has stronger in vitro AChE inhibition action than Donepezil. Furthermore, it has been found that, in vitro, Icopezil shows higher AChE selectivity than the existing AChE inhibitor tacrine, and clearly increases intracranial ACh in vivo (J. Med. Chem. 1995, 38, 2802-). Moreover, Zanapezil is known to have AChE inhibition action as well as monoamine nerve activation action, and is known as a drug having high central selectivity (67th Meeting of The Japanese Pharmacological Society, Neuropsychopharmacology 1994, 16, 729-). As hereinbefore described, the above compound is anticipated to be highly useful for amelioration of the symptoms of dementia, in the same way as Donepezil.

In JP, A, 11-315016 an ointment, etc. for percutaneous administration and a suppository for rectal administration are proposed as preparation forms suitable for dementia patients to take when symptoms are advanced and oral administration is difficult. Furthermore, it indicates that a base containing a higher alcohol and an ester derivative thereof can further improve the percutaneous absorbability of Donepezil hydrochloride. However, in this patent publication, although cases in which an ointment, a cream, and a suppository are used are cited as Examples, they are not practical for the continuous administration of an active ingredient over a long period of time and, furthermore, it can be expected that it would be difficult in practice to avoid the following undesirable situation that is inherent in an oral preparation.

That is, although the preparation form currently used clinically is an oral preparation, AChE inhibitors in the oral preparation form generally have strong side effects, and there are many reports relating, in particular, to liver malfunction, gastrointestinal disorders, etc. As causes therefor, it can be mentioned that it is generally difficult to prevent the first pass effect of an oral preparation in the liver, and the liver function is therefore easily affected; furthermore, since the oral preparation is present within the gastrointestinal tract at a high concentration, side effects can easily occur in the gastrointestinal tract. Furthermore, in the case of an oral preparation, with regard to changes in drug plasma concentration after administration, since the ratio (A/B) of the maximum plasma concentration (A) reached after administration to the plasma concentration (B) 24 hours after administration (at the time of a subsequent administration) is large, it is difficult to maintain the therapeutic effect over a long period of time without making the plasma concentration reach a concentration where side effects occur.

### Disclosure of Invention

It is therefore an object of the present invention to provide a percutaneous absorption preparation that contains as an active ingredient a drug such as Donepezil for the treatment of dementia and that can maintain the therapeutic effect over a long period of time without making the active ingredient plasma concentration reach a concentration where side effects occur.

As a result of an intensive investigation by the present inventors, it has been found that as long as a percutaneous absorption preparation has certain specific properties, there is not a transient rise in the plasma concentration of the drug for the treatment of dementia, and the drug for the treatment of dementia can be administered to a living body safely and effectively, and the present invention has thus been accomplished.

That is, the present invention relates to a percutaneous absorption preparation for the treatment of dementia, the preparation comprising an adhesive composition, the adhesive composition containing an active ingredient dispersed therein, the active ingredient being released at a pharmacologically effective rate, and the dermal penetration rate thereof being 1.2 µg/cm²/h or more.

Furthermore, the present invention relates to the above-mentioned preparation wherein the adhesive composition is capable of holding the percutaneous absorption preparation for the treatment of dementia on the skin surface for 12 hours or more.

Moreover, the present invention relates to the above-mentioned preparation wherein the ratio (A/B) of the maximum active ingredient plasma concentration (A) after administration to the active ingredient plasma concentration (B) 24 hours after administration is 1.3 or less.

Furthermore, the present invention relates to the above-mentioned preparation wherein the active ingredient is an acetylcholinesterase inhibitor.

Moreover, the present invention relates to the above-mentioned preparation wherein the acetylcholinesterase inhibitor is one or more selected from the group consisting of Donepezil, Zanapezil, Icopezil, a compound represented by the formula below, 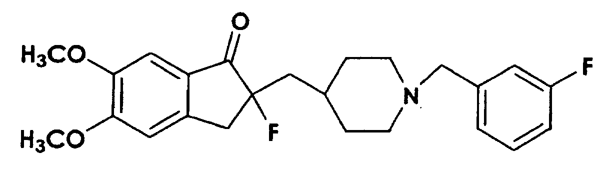 and pharmaceutically acceptable salts thereof.

Furthermore, the present invention relates to the above-mentioned preparation wherein the salt is one or more selected from the group consisting of a hydrochloride, a sulfate, a mesylate, a citrate, a fumarate, a tartrate, a maleate, and an acetate.

Moreover, the present invention relates to the above-mentioned preparation wherein the active ingredient is Donepezil hydrochloride.

Furthermore, the present invention relates to the above-mentioned preparation wherein the adhesive composition comprises a hydrophobic polymer and has self-adhesive power.

Moreover, the present invention relates to the above-mentioned preparation wherein the hydrophobic polymer is one or more selected from the group consisting of polyisoprene, polyisobutylene, polystyrene, polyethylene, polybutadiene, a styrene butadiene copolymer, a styrene-isoprene-styrene block copolymer, a styrene-butylene-styrene block copolymer, butyl rubber, natural rubber, a styrene-butadiene-styrene block copolymer, polysiloxane, and a (meth)acrylic acid polymer.

Furthermore, the present invention relates to the above-mentioned preparation wherein the hydrophobic polymer is polyisobutylene and/or a styrene-isoprene-styrene block copolymer.

Moreover, the present invention relates to the above-mentioned preparation wherein the hydrophobic polymer is a (meth)acrylic polymer formed by polymerization or copolymerization of one or more types of (meth)acrylate esters.

Furthermore, the present invention relates to the above-mentioned preparation wherein the (meth)acrylate ester is 2-ethylhexyl (meth)acrylate and/or butyl (meth)acrylate.

Moreover, the present invention relates to the above-mentioned preparation wherein the adhesive composition further comprises an absorption promoting agent for obtaining a therapeutically effective plasma concentration of the active ingredient.

Furthermore, the present invention relates to the above-mentioned preparation wherein the absorption promoting agent is one or more selected from the group consisting of lauric acid diethanolamide, sorbitan monolaurate, glycerol monolaurate, glycerol monooleate, glycerol monocaprate, glycerol monocaprylate, polyoxyethylene(4)-lauryl ether, and pyrrothiodecane.

Moreover, the present invention relates to the above-mentioned preparation wherein the adhesive composition further comprises one or more selected from the group consisting of organic acids and pharmaceutically acceptable salts thereof.

Furthermore, the present invention relates to the above-mentioned preparation wherein the organic acid is one or more selected from the group consisting of acetic acid, propionic acid, lactic acid, and salicylic acid.

Moreover, the present invention relates to a percutaneous absorption preparation for the treatment of dementia, the preparation comprising a hydrophobic matrix laminated between a support and a liner, wherein the hydrophobic matrix comprises an adhesive composition containing an active ingredient, the adhesive composition containing the active ingredient dispersed therein, the active ingredient is released at a pharmacologically effective rate, and the skin permeation rate thereof is 1.2 µg/cm²/h or more.

Since the percutaneous absorption preparation for the treatment of dementia of the present invention has the above-mentioned constitutions, it can continuously release a drug for the treatment of dementia while maintaining a substantially constant skin permeation rate without transiently increasing the plasma concentration of the drug for the treatment of dementia, and can be administered over a long period of time.

### Brief Description of the Drawings

FIG. 1 is a cross-sectional view showing one embodiment of the percutaneous absorption preparation of the present invention.
FIG. 2 is a graph showing the result of an in vitro human skin permeation test using the percutaneous absorption preparation of the present invention.
FIG. 3 is a graph showing the human plasma concentration of active ingredient from a single administration of the percutaneous absorption preparation of the present invention.
FIG. 4 is a graph showing the human plasma concentration of active ingredient when the percutaneous absorption preparation of the present invention is administered continuously.

### Modes for Carrying Out the Invention

The percutaneous absorption preparation for the treatment of dementia of the present invention is explained further in detail below.

The percutaneous absorption preparation referred to in the present description means an adhesive preparation containing at least a support and an adhesive composition and, generally speaking, this includes a reservoir type external adhesive preparation and a matrix type external adhesive preparation. Comparing the reservoir type external adhesive preparation with the matrix type external adhesive preparation, in general, the matrix type external adhesive preparation, in which an adhesive composition having self-adhesive power is adhered directly to the skin, has excellent adhesion properties and gives excellent drug absorbability, and the percutaneous absorption preparation of the present invention is explained below using mainly the matrix type adhesive preparation as an example, but the present invention should not be construed as being limited thereto.

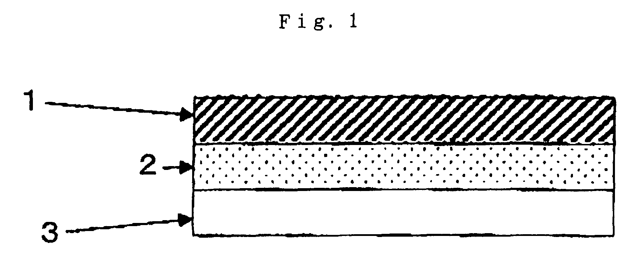

The configuration of the percutaneous absorption preparation for the treatment of dementia of the present invention is not particularly limited as long as the adhesive composition includes the active ingredient dispersed therein, the active ingredient is released at a pharmacologically effective rate, and the dermal penetration rate thereof is 1.2 µg/cm²/h or more. A typical configuration includes a hydrophobic matrix (adhesive layer) containing a medicament (drug for the treatment of dementia), and a support on the reverse side thereof as shown in FIG. 1. This adhesive layer preferably has an adhesive power that is sufficient to maintain a therapeutically acceptable, effective area on the skin surface for 12 hours or more, but if this is difficult, it is also possible to use a cover sheet having adhesive power and an area that is larger than the medicament-containing layer.

The percutaneous absorption preparation of the present invention enables stable supply of a medicament without any problem of adhesion by having the drug for the treatment of dementia and/or a pharmaceutically acceptable salt thereof dissolved or dispersed in the adhesive composition.

The drug for the treatment of dementia that can be used in the present invention is not particularly limited as long as it exhibits an anti-dementia effect, and examples thereof include Donepezil, Icopezil (CP-118954, 5,7-dihydro-3-[2-[1-(phenylmethyl)-4-piperidinyl]ethyl]-6H-pyrrolo-[4,5-f]-1,2-benzisoxazol-6-one maleate), ER-127528 (4-[(5,6-dimethoxy-2-fluoro-1-indanon)-2-yl]methyl-1-(3-fluorobenzyl)piperidine hydrochloride), TAK-147 (3-[1-(phenylmethyl)piperidin-4-yl]-1-(2,3,4,5-tetrahydro-1H-2-benzazepin-8-yl)-1-propane fumarate), tacrine, Physostigmine, Neostigmine, Rivastigmine, Galantamine, Metrifonate, T-588 ((-)-R-α-[[2-(dimethylamino)ethoxy]methyl]benzo[b]thiophene-5-methanol hydrochloride), FK-960 (*N*-(4-acetyl-1-piperazinyl)-*p*-fluorobenzamide-hydrate), TCH-346 (*N*-methyl-*N*-2-pyropinyldibenz[b,f]oxepine-10-methanamine), SDZ-220-581 ((S)-α-amino-5-(phosphonomethyl)-[1,1'-biphenyl]-3-propionic acid), EAB-318, SM-31900, and pharmaceutically acceptable salts thereof, and these drugs for the treatment of dementia can be used singly or in a combination of two or more types in a pharmaceutically acceptable range.

The type of salt is not particularly limited, but a hydrochloride, a sulfate, a mesylate, a citrate, a fumarate, a tartrate, a maleate, or an acetate is preferable.

Among these drugs for the treatment of dementia, Donepezil hydrochloride is particularly preferable.

Donepezil hydrochloride, which is generally used as an oral anti-dementia drug, is administered at a usual dose of 5 mg once a day. That is, use of such an amount as an oral preparation is necessary for exhibiting the efficacy of Donepezil.

However, even if the plasma concentration of a medicament when used as an oral preparation is clarified, when it is used for percutaneous absorption, since the skin penetrability is completely different from one medicament to another, it is not easy to infer a dermal penetration rate that can give a plasma concentration that exhibits substantially the same efficacy as in the case of oral administration.

Under such circumstances, the present inventors have found that, while taking the pharmacokinetics of Donepezil into consideration, in order to maintain a therapeutically effective plasma concentration of Donepezil, it is necessary for the absorption rate to be 120 µg/h or more and, furthermore, have invented a percutaneous absorption preparation having an adhesive composition that can achieve the target value.

An absorption rate of 120 µg/h depends on the area of the skin to which the percutaneous absorption preparation is applied; here, it is considered that a normal percutaneous absorption preparation has an area of 100 cm² or less, and the present inventors have accomplished the present invention by producing a percutaneous absorption preparation having a dermal penetration rate for the drug for the treatment of dementia of 1.2 µg/cm²/h or more.

As one embodiment of the percutaneous absorption preparation of the present invention, with regard to the change in plasma concentration after human administration, the ratio (A/B) of the maximum plasma concentration (A) after administration of the preparation to the plasma concentration (B) 24 hours after administration is 1.3 or less in the case of either a single administration or continuous administration. This can prevent the occurrence of side effects due to a transient increase in the plasma concentration of Donepezil, which is observed for oral administration. That is, the percutaneous absorption preparation of the present invention gradually increases the plasma concentration of Donepezil, the efficacy can be exhibited sufficiently over a long period of time thereafter, and the plasma concentration of the drug can be maintained at a level at which side effects do not occur.

With regard to the adhesive composition of the present invention, the adhesive composition having self-adhesive power contains a hydrophobic polymer. This hydrophobic polymer is not particularly limited, but an acrylic polymer or a rubber polymer is preferably used.

The acrylic polymer is not particularly limited as long as it is a copolymer of at least one type of a (meth)acrylic acid derivative represented by 2-ethylhexyl acrylate, methyl acrylate, butyl acrylate, hydroxyethyl acrylate, and 2-ethylhexyl methacrylate, and examples thereof are described as adhesives in Iyakuhin Tenkabutsu Jiten (Pharmaceutical Excipients Dictionary) 2000 (Ed. by Japan Pharmaceutical Excipients Council) and include an acrylic acid/octyl acrylate copolymer, a 2-ethylhexyl acrylate/vinylpyrrolidine copolymer solution, an acrylate ester/vinyl acetate copolymer, a 2-ethylhexyl acrylate/2-ethylhexyl methacrylate/dodecyl methacrylate copolymer, a methyl acrylate/2-ethylhexyl acrylate copolymer resin emulsion, an adhesive such as an acrylic polymer contained in an acrylic resin alkanolamine solution, the DURO-TAK acrylic adhesive series (manufactured by National Starch and Chemical Company), and the EUDRAGIT series (Higuchi Shokai Co., Ltd.).

Examples of the rubber polymer include a styrene-isoprene-styrene block copolymer (hereinafter abbreviated to SIS), isoprene rubber, polyisobutylene (hereinafter abbreviated to PIB), a styrene-butadiene-styrene block copolymer (hereinafter abbreviated to SBS), styrenebutadiene rubber (hereinafter abbreviated to SBR), and polysiloxane, and thereamong, SIS and PIB are preferable, and SIS is particularly preferable.

Such hydrophobic polymers can be used in a combination of two or more types and, while taking into consideration the formation of an adhesive layer and adequate penetrability, the amounts of these polymers added relative to the weight of the entire composition is 5 to 90 wt %, preferably 10 to 70 wt %, and more preferably 10 to 50 wt %.

In the percutaneous absorption preparation of the present invention, when the form of the medicament is a pharmaceutically acceptable acid addition salt, it is preferable for the adhesive layer to further contain an organic acid and/or a pharmaceutically acceptable salt thereof. The organic acid that can be used is not particularly limited, and examples thereof include aliphatic (mono-, di-, and tri-) carboxylic acids (e.g., acetic acid, propionic acid, isobutyric acid, caproic acid, caprylic acid, lactic acid, maleic acid, pyruvic acid, oxalic acid, succinic acid, tartaric acid, etc.), aromatic carboxylic acids (e.g., phthalic acid, salicylic acid, benzoic acid, acetylsalicylic acid, etc.), alkylsulfonic acids (e.g., methanesulfonic acid, ethanesulfonic acid, propylsulfonic acid, butanesulfonic acid, polyoxyethylene alkyl ether sulfonic acids, etc.), alkylsulfonic acid derivatives (e.g., *N*-2-hydroxyethylpiperidine-N'-2-ethanesulfonic acid (hereinafter abbreviated to 'HEPES'), etc.), and cholic acid derivatives (e.g., dehydrocholic acid, etc.) and, thereamong, acetic acid, propionic acid, lactic acid, and salicylic acid are preferable, and acetic acid is particularly preferable. These organic acids can be salts thereof or a mixture with a salt thereof. Addition of such an organic acid to the adhesive composition enables the skin penetrability to be enhanced.

In particular, when Donepezil hydrochloride is used, by adding an acetate salt to the adhesive composition, the above-mentioned adequate dermal penetration rate can be obtained. Similarly, when the hydrochloride of ER-127528, the maleate of Icopezil, or the fumarate of Zanapezil is used as the medicament, by adding an acetate salt to the adhesive composition, the skin permeability can be enhanced.

Taking into consideration an adequate level of penetration and irritation of the skin by the adhesive preparation, the amount of these organic acids added relative to the weight of the entire composition of the adhesive layer is preferably 0.01 to 20 wt %, more preferably 0.1 to 15 wt %, and particularly preferably 0.1 to 10 wt %.

The adhesive composition of the percutaneous absorption preparation of the present invention can contain an absorption enhancing agent; the absorption enhancing agent that can be used here can be any compound that is conventionally recognized to have a skin absorption enhancing effect, and examples thereof include fatty acids, fatty alcohols, fatty acid esters, amides, and ethers having 6 to 20 carbon chains, aromatic organic acids, aromatic alcohols, aromatic organic acid esters and ethers (those above can be either saturated or unsaturated, and can be cyclic, straight chain, or branched) and, furthermore, lactate esters, acetate esters, monoterpenoid compounds, sesquiterpenoid compounds, Azone, Azone derivatives, pyrrothiodecane, glycerol fatty acid esters, propylene glycol fatty acid esters, sorbitan fatty acid esters (Span series) polysorbate types (Tween series), polyethylene glycol fatty acid esters, polyoxyethylene hardened castor oil types (HCO series), polyoxyethylene alkyl ethers, sucrose fatty acid esters, and vegetable oils.

Specifically, preferred examples include caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrrothiodecane, and olive oil, and particularly preferred examples include lauryl alcohol, isostearyl alcohol, lauric acid diethanolamide, glycerol monocaprylate, glycerol monocaprate, glycerol monooleate, sorbitan monolaurate, propylene glycol monolaurate, polyoxyethylene lauryl ether, and pyrrothiodecane.

Such absorption promoting agents can be used in a combination of two or more types and, while taking into consideration adequate permeability as an adhesive preparation and irritation of the skin such as reddening or edema, they can preferably be added at 0.01 to 20 wt %, more preferably 0.05 to 10 wt %, and particularly preferably 0.1 to 5 wt %, relative to the weight of the entire composition of the adhesive layer.

It is also possible to add a plasticizer to the percutaneous absorption adhesive composition of the present invention. Examples of the plasticizer that can be used include petroleum oil (e.g., paraffinic process oil, naphthenic process oil, aromatic process oil, etc.), squalane, squalene, plant oil (e.g., olive oil, camellia oil, castor oil, tall oil, peanut oil), silicon oil, dibasic acid esters (e.g., dibutyl phthalate, dioctyl phthalate, etc.), liquid rubber (e.g., polybutene, liquid isoprene rubber), liquid fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, and crotamiton. In particular, liquid paraffin, liquid polybutene, isopropyl myristate, diethyl sebacate, and hexyl laurate are preferable.

These components can be used as a mixture of two or more types and, taking into consideration the maintenance of adequate permeability and adequate cohesion as an adhesive preparation, the amount of such a plasticizer added relative to the entire composition of the adhesive layer can be 10 to 70 wt %, preferably 10 to 60 wt %, and more preferably 10 to 50 wt %.

When the adhesive power of the adhesive layer of the present invention is not sufficient to maintain 12 hours or more of application, it is preferable for the adhesive layer to contain an adhesion-imparting resin, and examples of the adhesion-imparting resin that can be used include rosin derivatives (e.g., rosin, a glycerol ester of rosin, hydrogenated rosin, a glycerol ester of hydrogenated rosin, a pentaerythritol ester of rosin, etc.), saturated alicyclic hydrocarbon resins (e.g., Arkon P100, manufactured by Arakawa Chemical Industries, Ltd.), aliphatic hydrocarbon resins (e.g., Quintone B170, manufactured by Nippon Zeon Corporation), terpene resins (e.g., Clearon P-125, manufactured by Yasuhara Chemical Co., Ltd.), and maleic acid resins. In particular, a glycerol ester of hydrogenated rosin, a saturated alicyclic hydrocarbon resin, an aliphatic hydrocarbon resin, and a terpene resin are preferable.

Taking into consideration adequate adhesive power and irritation of the skin during removal of the adhesive preparation, the amount of such an adhesion-imparting resin added relative to the entire composition of the adhesive composition can be 5 to 70 wt %, preferably 5 to 60 wt %, and more preferably 10 to 50 wt %.

Moreover, as necessary, an antioxidant, a filler, a crosslinking agent, a preservative, or an ultraviolet-absorbing agent can be used, and preferred examples of the antioxidant include tocopherol and ester derivatives thereof, ascorbic acid, ascorbyl stearate, nordihydroguaiaretic acid, dibutyl hydroxytoluene (BHT), and butyl hydroxyanisole. Preferred examples of the filler include calcium carbonate, magnesium carbonate, silicate salts (e.g., aluminum silicate, magnesium silicate, etc.), silicic acid, barium sulfate, calcium sulfate, calcium zincate, zinc oxide, and titanium oxide. Preferred examples of the crosslinking agent include thermosetting resins such as amino resins, phenolic resins, epoxy resins, alkyd resins, and unsaturated polyesters, organic crosslinking agents such as isocyanate compounds and blocked isocyanate compounds, and inorganic crosslinking agents such as metals and metal compounds. Preferred examples of the preservative include ethyl paraoxybenzoate, propyl paraoxybenzoate, and butyl paraoxybenzoate. Preferred examples of the ultraviolet-absorbing agent include p-aminobenzoic acid derivatives, anthranilic acid derivatives, salicylic acid derivatives, coumarin derivatives, amino acid compounds, imidazoline derivatives, pyrimidine derivatives, and dioxane derivatives.

The total amount of such antioxidant, filler, crosslinking agent, preservative, and ultraviolet-absorbing agent can be preferably 10 wt % or less, more preferably 5 wt % or less, and particularly preferably 2 wt % or less, relative to the weight of the entire composition of the adhesive layer of the adhesive preparation.

A medicament-containing adhesive layer having the above-mentioned composition can be produced by any method. For example, a medicament-containing base composition is melted by heating, applied on a release paper or a support, and then laminated to a support or a release paper to give the present preparation. Alternatively, a medicament-containing base component is dissolved in a solvent such as toluene, hexane, or ethyl acetate and spread on a release paper or a support, and after the solvent is removed by drying, the medicament-containing base component is laminated to a support or a release paper to give the present preparation.

The percutaneous absorption preparation of the present invention is typically a percutaneous absorption preparation shown in FIG. 1, and a stretchable or non-stretchable support can be employed as the support. For example, it can be selected from fabrics, nonwoven fabrics, polyurethane, polyester, polyvinyl acetate, polyvinylidene chloride, polyethylene, polyethylene terephthalate, aluminum sheet, and composite materials thereof.

Furthermore, a liner is not particularly limited as long as it can protect the adhesive layer until the percutaneous absorption preparation is applied to the skin, does not degrade the drug for the treatment of dementia, and is coated with a silicon coating so as to ensure easy release, and specific examples thereof include a silicon-coated polyethylene film, polyethylene terephthalate film, and polypropylene film.

### Examples

The present invention is explained below in further detail with reference to Examples of the present invention, but the Examples should not be construed as limiting the present invention, and the present invention can be modified in a variety of ways without departing from the technical scope thereof. In the Examples, '%' denotes 'wt %' unless otherwise specified.

### Example 1

| (Formulation) | |
|---|---|
| SIS | 17.1% |
| PIB | 7.3% |
| Saturated alicyclic hydrocarbon resin (Arkon P100) | 41.6% |
| Liquid paraffin | 29.4% |
| Sodium acetate | 0.6% |
| Donepezil hydrochloride | 1.0% |
| Pyrrothiodecane | 3.0% |
| Total | 100.0% |

The Donepezil hydrochloride, sodium acetate, pyrrothiodecane, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Example 2

| (Formulation) | |
|---|---|
| SIS | 16.6% |
| Acrylic polymer | 7.0% |
| (Duro-Tak 87-2287, National Starch & Chemicals) Saturated alicyclic hydrocarbon resin (Arkon P100) | 40.2% |
| Liquid paraffin | 28.4% |
| Sodium acetate | 1.8% |
| Donepezil hydrochloride | 3.0% |
| Pyrrothiodecane | 3.0% |
| Total | 100.0% |

The Donepezil hydrochloride, sodium acetate, pyrrothiodecane, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Example 3

| (Formulation) | |
|---|---|
| SIS | 16.0% |
| PIB | 6.8% |
| Saturated alicyclic hydrocarbon resin (Arkon P100) | 38.8% |
| Liquid paraffin | 27.4% |
| Sodium acetate | 3.0% |
| Donepezil hydrochloride | 5.0% |
| Sorbitan monolaurate | 3.0% |
| Total | 100.0% |

The Donepezil hydrochloride, sodium acetate, sorbitan monolaurate, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Example 4

| (Formulation) | |
|---|---|
| SIS | 16.0% |
| PIB | 6.9% |
| Saturated alicyclic hydrocarbon resin (Arkon P100) | 38.9% |
| Liquid paraffin | 27.5% |
| Sodium acetate | 2.7% |
| ER-127528 | 5.0% |
| Pyrrothiodecane | 3.0% |
| Total | 100.0% |

The ER-127528, sodium acetate, pyrrothiodecane, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Example 5

| (Formulation) | |
|---|---|
| SIS | 16.1% |
| PIB | 6.9% |
| Saturated alicyclic hydrocarbon resin (Arkon P100) | 39.0% |
| Liquid paraffin | 27.5% |
| Sodium acetate | 2.5% |
| Zanapezil | 5.0% |
| Pyrrothiodecane | 3.0% |
| Total | 100.0% |

The Zanapezil (TAK-147), sodium acetate, pyrrothiodecane, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Example 6

| (Formulation) | |
|---|---|
| SIS | 16.1% |
| PIB | 6.9% |
| Saturated alicyclic hydrocarbon resin (Arkon P100) | 39.0% |
| Liquid paraffin | 27.5% |
| Sodium acetate | 2.5% |
| Icopezil | 1.0% |
| Pyrrothiodecane | 3.0% |
| Total | 100.0% |

The Icopezil (CP-118954), sodium acetate, pyrrothiodecane, and liquid paraffin were weighed in a mortar and mixed well in advance, and then mixed with the rest of the components dissolved in toluene. After the mixture was applied onto a release paper, the solvent was removed by drying, and a PET film support was laminated thereto, thus giving a percutaneous absorption preparation of the present invention.

### Comparative Examples 1 to 3

By not adding sodium acetate to the percutaneous absorption preparations of Examples 1 to 3, the corresponding percutaneous absorption preparations of Comparative Examples 1 to 3 were thus obtained. Apart from the sodium acetate not being added, the same components and the same procedure for the preparation process of Examples 1 to 3 were employed.

### Test Example 1 Hairless mouse skin penetration test

Hairless mouse dorsal skin was peeled off and mounted on a flow-through cell (5 cm²), through the outer periphery of which warm water at 37°C was circulating, so that the dermis side was on the receptor layer side. Each of the preparations obtained in Examples 1 to 6 and Comparative Examples 1 to 3 was adhered to the corneum layer side, and sampling was carried out using physiological saline at 5 ml/hour on the receptor layer every 2 hours up to 24 hours. With regard to the receptor solution obtained at each time, the flow rate was measured precisely and the medicament concentration was measured by high performance liquid chromatography. The penetration rate per hour was calculated from the flow rate and the medicament concentration, and the maximum dermal penetration rate for each of the Examples was determined.

The results for the preparations of Examples 1 to 3 and Comparative Examples 1 to 3 are shown in Table 1, and the results for the preparations of Examples 4 to 6 are shown in Table 2.

### Test Example 2 Rabbit primary skin irritation test

A primary skin irritation test for the preparations obtained in Examples 1 to 3 was carried out by the Draize method. The PII values obtained for each of the preparations are shown in Table 1.

### Test Example 3 Test of physical properties of preparation

The preparations obtained in Examples 1 to 6 were subjected to measurement of adhesive power using a probe tack tester and a peel tester, and measurement of cohesion using a creep tester. Stringiness, exudation of the solution components, etc. were examined visually. The results were evaluated as 'good' when there was no problem with the physical properties of the preparation, and 'poor' when there was a problem.

The results for the preparations of Examples 1 to 3 are shown in Table 1. The results for the preparations of Examples 4 to 6 are shown in Table 2.

**Table 1**

| | Permeation rate (µg/cm²/hour) | PII value | Physical properties |
|---|---|---|---|
| Example 1 | 6.4 | 0.8 | Good |
| Example 2 | 14.0 | 0.9 | Good |
| Example 3 | 18.8 | 0.9 | Good |
| Comp. Ex. 1 | 0.1 | - | - |
| Comp. Ex. 2 | 0.3 | - | - |
| Comp. Ex. 3 | 0.4 | - | - |
| - : No data | | | |

**Table 2**

| | Permeation rate (µg/cm²/hour) | Physical properties |
|---|---|---|
| Example 4 | 1.6 | Good |
| Example 5 | 4.2 | Good |
| Example 6 | 2. 7 | Good |

### Test Example 4 In vitro human skin permeation test

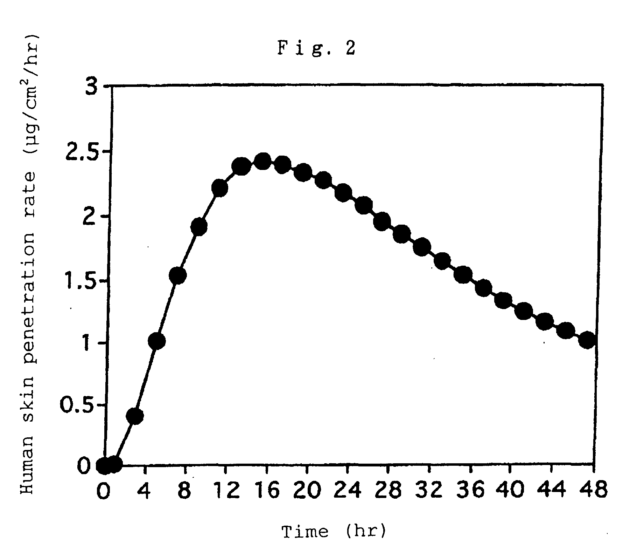

Human skin, which had been dermatomed to about 500 µm, was mounted on a flow-through cell (5 cm²), through the outer periphery of which warm water at 33°C was circulating, so that the dermis side was arranged on the receptor layer side. The preparation obtained in Example 1 was adhered to the corneum layer side, and sampling was carried out using physiological saline at 5 ml/hour on the receptor layer every 2 hours up to 48 hours. With regard to the receptor solution obtained at each time, the flow rate was measured precisely, the medicament concentration was measured by high performance liquid chromatography, and the permeation rate per hour was calculated. The results are shown in FIG. 2.

### Test Example 5 Method for calculating human plasma concentration profile

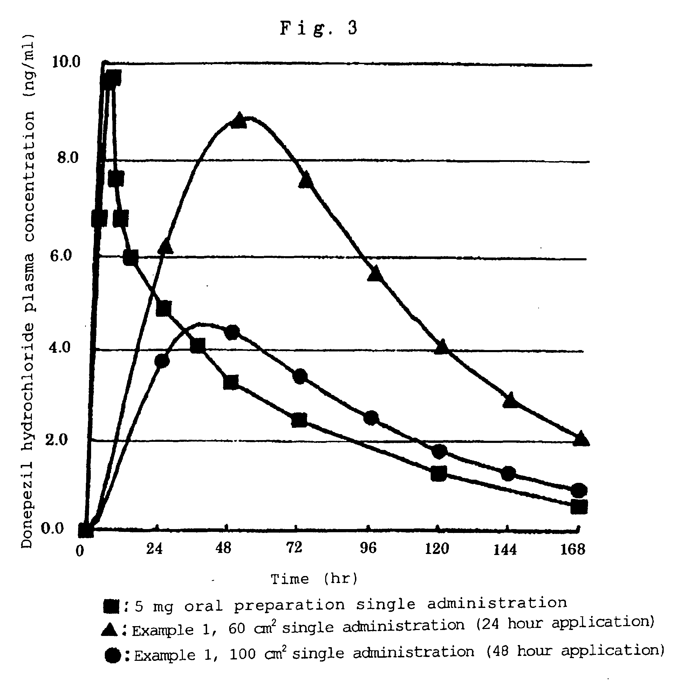

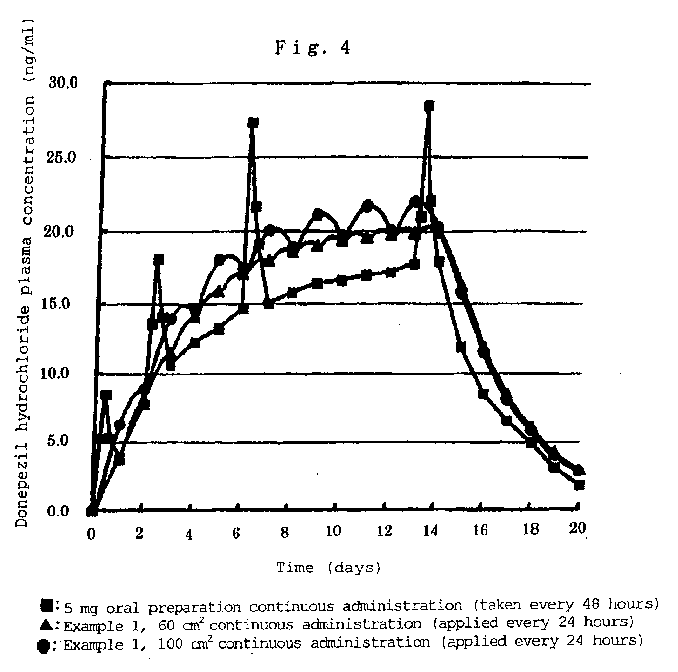

The pharmacokinetic parameters of Donepezil hydrochloride for human oral administration were determined by the pharmacokinetic analysis software WinNonlin (Scientific Consulting. Inc.) using known data for an oral preparation (5 mg). Human plasma concentrations were calculated for a single administration and for continuous administration (2 week continuous administration) by the percutaneous absorption prediction system SKIN-CAD™ Professional Edition ver.1.1 (i-Hive Communication Inc.) using the above parameters and the results of the human skin permeation test obtained in Test Example 4 (FIG. 2). The results are shown in FIG. 3 and FIG. 4. The preparation area was 60 cm² in the case of 24 hour administration and 100 cm² in the case of 48 hour administration. For comparison, the plasma concentration of the 5 mg oral preparation is also shown.

It has been clearly found from the above-mentioned results that the Donepezil hydrochloride-containing adhesive preparations obtained in the Examples of the present invention have an outstandingly high medicament skin permeation rate, and the skin irritation and physical properties of the preparations are fully acceptable for practical application.

### Industrial Applicability

In accordance with the percutaneous absorption preparation of the present invention, a drug for the treatment of dementia such as Donepezil hydrochloride can be absorbed efficiently through the skin into circulating blood. Furthermore, it is possible to avoid side effects in the gastrointestinal tract, which occur in the case of oral administration, and side effects in the central system, which are due to a rapid increase in blood concentration. Moreover, adhesion to the skin is good, and it is particularly effective as an external preparation for the purpose of percutaneous application.

## Claims

1. A percutaneous absorption preparation for the treatment of dementia, the preparation comprising an adhesive composition, the adhesive composition containing an active ingredient dispersed therein, the active ingredient being released at a pharmacologically effective rate, and the skin permeation rate thereof being 1.2 µg/cm²/h or more.

2. The preparation according to Claim 1, wherein the adhesive composition is capable of holding the percutaneous absorption preparation for the treatment of dementia on the skin surface for 12 hours or more.

3. The preparation according to either Claim 1 or 2, wherein the ratio (A/B) of the maximum active ingredient plasma concentration (A) after administration to the active ingredient plasma concentration (B) 24 hours after administration is 1.3 or less.

4. The preparation according to any one of Claims 1 to 3, wherein the active ingredient is an acetylcholinesterase inhibitor.

5. The preparation according to Claim 4, wherein the acetylcholinesterase inhibitor is one or more selected from the group consisting of Donepezil, Zanapezil, Icopezil, a compound represented by the formula below, 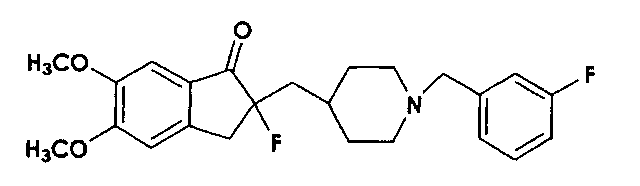 and pharmaceutically acceptable salts thereof.

6. The preparation according to Claim 5, wherein the salt is one or more selected from the group consisting of a hydrochloride, a sulfate, a mesylate, a citrate, a fumarate, a tartrate, a maleate, and an acetate.

7. The preparation according to Claim 6, wherein the active ingredient is Donepezil hydrochloride.

8. The preparation according to any one of Claims 1 to 7, wherein the adhesive composition comprises a hydrophobic polymer and has self-adhesive power.

9. The preparation according to Claim 8, wherein the hydrophobic polymer is one or more selected from the group consisting of polyisoprene, polyisobutylene, polystyrene, polyethylene, polybutadiene, a styrene butadiene copolymer, a styrene-isoprene-styrene block copolymer, a styrene-butylene-styrene block copolymer, butyl rubber, natural rubber, a styrene-butadiene-styrene block copolymer, polysiloxane, and a (meth)acrylic acid polymer.

10. The preparation according to Claim 9, wherein the hydrophobic polymer is polyisobutylene and/or a styrene-isoprene-styrene block copolymer.

11. The preparation according to Claim 9, wherein the hydrophobic polymer is a (meth)acrylic polymer formed by polymerization or copolymerization of one or more types of (meth)acrylate esters.

12. The preparation according to Claim 11, wherein the (meth)acrylate ester is 2-ethylhexyl (meth)acrylate and/or butyl (meth)acrylate.

13. The preparation according to any one of Claims 1 to 12, wherein the adhesive composition further comprises an absorption promoting agent for obtaining a therapeutically effective plasma concentration of the active ingredient.

14. The preparation according to Claim 13, wherein the absorption promoting agent is one or more selected from the group consisting of lauric acid diethanolamide, sorbitan monolaurate, glycerol monolaurate, glycerol monooleate, glycerol monocaprate, glycerol monocaprylate, polyoxyethylene(4)-lauryl ether, and pyrrothiodecane.

15. The preparation according to any one of Claims 1 to 14, wherein the adhesive composition further comprises one or more selected from the group consisting of organic acids and pharmaceutically acceptable salts thereof.

16. The preparation according to Claim 10, wherein the organic acid is one or more selected from the group consisting of acetic acid, propionic acid, lactic acid, and salicylic acid.

17. A percutaneous absorption preparation for the treatment of dementia, the preparation comprising a hydrophobic matrix laminated between a support and a liner, wherein the hydrophobic matrix comprises an adhesive composition containing an active ingredient, the adhesive composition containing the active ingredient dispersed therein, the active ingredient is released at a pharmacologically effective rate, and the skin permeation rate thereof is 1.2 µg/cm²/h or more.
